# EUROPÄISCHE PATENTANMELDUNG

(11) **EP 3 385 393 A1**
(43) Veröffentlichungstag der Anmeldung: **10.10.2018**
(21) Anmeldenummer: 17165126.8
(22) Anmeldetag: 05.04.2017
(51) Int. Cl.: C12Q 1/68

(54) **IN-VITRO-VERFAHREN ZUR DIAGNOSE DES RISIKOS EINER PERSON ZUR AUSBILDUNG EINES AEROTOXISCHEN SYNDROMS UND KIT ZUR DURCHFÜHRUNG DES VERFAHRENS**

(71) Anmelder: Schnakenberg, Eckart, 28832 Achim (DE)
(72) Erfinder: Schnakenberg, Eckart, 28832 Achim (DE)
(74) Vertreter: Pfenning, Meinig & Partner mbB

(57) **Zusammenfassung**

Es wird ein *in-vitro*-Verfahren zur Diagnose des Risikos einer Person zur Ausbildung eines Aerotoxischen Syndroms bereitgestellt. Das Verfahren basiert darauf, dass eine Probe einer Person mit einem molekulargenetischen Verfahren untersucht wird, wobei durch die Untersuchung bestimmt wird, ob mindestens ein Gen kodierend für Butyrylcholinesterase, Cytochrom P450 2D6 und/oder Cytochrom P450 2C9 homozygot mindestens eine Sequenzvariante oder heterozygot mindestens zwei Sequenzvarianten aufweist. Wenn das mindestens eine Gen homozygot mindestens eine Sequenzvariante oder heterozygot mindestens zwei Sequenzvarianten aufweist, wird die Person als anfällig eingestuft, ein Aerotoxisches Syndrom zu entwickeln. Ferner kann durch das Verfahren ein akutes Aerotoxisches Syndrom diagnostiziert werden, wenn die Person akut an Beschwerden leidet, die für ein Aerotoxisches Syndrom typisch sind. Ferner wird ein Kit zur Durchführung des erfindungsgemäßen Verfahrens bereitgestellt.

## Beschreibung

Es wird ein *in-vitro*-Verfahren zur Diagnose des Risikos einer Person zur Ausbildung eines Aerotoxischen Syndroms bereitgestellt. Das Verfahren basiert darauf, dass eine Probe einer Person mit einem molekulargenetischen Verfahren untersucht wird, wobei durch die Untersuchung bestimmt wird, ob mindestens ein Gen kodierend für Butyrylcholinesterase, Cytochrom P450 2D6 und/oder Cytochrom P450 2C9 homozygot mindestens eine Sequenzvariante oder heterozygot mindestens zwei Sequenzvarianten aufweist. Wenn das mindestens eine Gen homozygot mindestens eine Sequenzvariante oder heterozygot mindestens zwei Sequenzvarianten aufweist, wird die Person als anfällig eingestuft, ein Aerotoxisches Syndrom zu entwickeln. Ferner kann durch das Verfahren ein akutes Aerotoxisches Syndrom diagnostiziert werden, wenn die Person akut an Beschwerden leidet, die für ein Aerotoxisches Syndrom typisch sind. Ferner wird ein Kit zur Durchführung des erfindungsgemäßen Verfahrens bereitgestellt.

Trikresylphosphat (TCP) ist ein Organophosphat, das als Zusatzstoff in Öl- und Hydraulikflüssigkeiten von Flugzeugen verwendet wird. Bei Erhitzung dieser Flüssigkeiten entstehen Trikresylphosphat-haltige Öldämpfe, die nachgewiesenermaßen eine hohe Toxizität besitzen. Trikresyl-haltige Gase werden insbesondere durch Verbrennung von Hydrauliköl in Flugzeugen freigesetzt und können in die Kabinen des Flugzeugs gelangen, wo sie vom Flugpersonal und von den Passagieren über die Atemluft aufgenommen werden können. Das Freisetzungsereignis an sich wird als sog. 'fume event' bezeichnet.

Durch die Exposition mit Trikresylphosphat-haltigen Öldämpfen kann es akut und/oder mittelfristig zu gesundheitlichen Problemen kommen, welche als Aerotoxisches Syndrom beschrieben werden. Es ist somit bekannt, dass das Aerotoxische Syndrom durch Trikresylphoshphat-haltige Gase ausgelöst wird.

Bemerkenswert ist jedoch, dass bei gleich starker Exposition gegenüber Trikresyl-haltigem Gas nicht jeder Mensch bzw. Passagier im Flugzeug am Aerotoxischen Syndrom erkrankt. Da nicht jeder Exponierte gleichermaßen an definierten Symptomen erkrankt, gibt es eine individuelle genetisch bedingte Suszeptibilität für dieses Krankheitsbild. Gemäß der Arbeitsmedizinischen Leitlinie wird TCP als sehr giftig eingestuft (Arbeitsmedizinische S1-Leitlinie 002/022: Arbeiten unter Einwirkung von organischen Phosphorverbindungen, AWMF-Register Nr. 002/022, Klasse: S1, Deutsche Gesellschaft für Arbeitsmedizin und Umweltmedizin, Stand: 07/2014, siehe http://www.awmf.org/uploads/tx_szleitlinien/002-022l_S1_Organische_Phosphorverbindungen_2014-07.pdf).

Bei Exposition mit TCP ist es möglich, dass klinisch relevante Symptome sofort und/oder nach mehrmaligen, aufeinander folgenden Expositionen auftreten. Beide Situationen können mit ernsthaften Folgen für den Flugverkehr verbunden sein. In Körperflüssigkeiten (Blut bzw. Urin) ist TCP bzw. seine Abbauprodukte nur maximal 24 Stunden nach Beendigung einer Flug-bedingten Exposition nachweisbar. Dies stellt ein besonderes Problem zum Nachweis eines Aerotoxischen Syndroms der betroffenen Personen dar, denn häufig ist ein Test auf TCP in diesem Zeitraum nach einem Flug nicht möglich. Darüberhinaus klagen viele Patienten über neurologische Symptome, aber können diese nicht mit der Exposition mit Trikresyl-haltigen Gasen in Verbindung bringen. Grund hierfür ist, dass es nur selten zu sichtbaren (nebeligen) Dämpfen im Kabineninnenraum des Flugzeuges kommt. Ferner führt die Exposition mit Trikresyl-haltigen Gasen nicht bei jeder Person unmittelbar (akut) zu Beschwerden. Oft zeigen sich die Beschwerden erst nach einer jahrelangen, wiederkehrenden Exposition. Ferner kann die durch TCP ausgelöste gesundheitliche Beeinträchtigung über Stunden, Tagen, Wochen oder sogar länger anhalten. Nach wiederkehrenden TCP-Expositionen kann es gegebenenfalls zur Arbeitsunfähigkeit kommen.

Der Stoffwechsel von TCP im menschlichen Organismus ist im Einzelnen wenig bekannt. Die TCP-Exposition aus der Zapfluft von Flugzeugen ist eine Exposition mit einem Isomerengemisch von TCP. Der tatsächliche TCP-Metabolismus ist individuell unterschiedlich und kann mit In-vivo-Untersuchungen nur ansatzweise erklärt werden. Grundsätzlich entstehen bei der Umwandlung von Organophosphaten wie TCP Metabolite, die ein größeres toxisches Potenzial haben als die Ausgangssubstanzen selbst. Es ist bekannt, dass Organophosphate in Pestiziden und Kampfstoffen potente Inhibitoren der Acetylcholinesterase sind und zu einer Organophosphat-induzierten verzögerten Neuropathie (OPIDN) beitragen können (Weiner und Jortner, Organophosphate-induced delayed neurotoxicity of triarylphosphates, Neurotoxicology, 20(4): 653-673, 1999).

Neben Expositionen gegenüber Organophosphaten aus Hydraulikölen in Flugzeugen wurden Organophosphate auch in der Umwelt nachgewiesen und stellen eine ,Hintergrundbelastung' aus der Umwelt für nicht-beruflich Exponierte dar. Es wird vermutet, dass es neben einer inhalativen (pulmonalen) Aufnahme von TCP auch zu einer transdermalen Resorption von TCP kommen kann. Der Hauptausscheidungsweg von TCP verläuft über den Urin. Im Tierversuch konnten als Abbauprodukte von TCP das neurotoxische Saligenin-o-tolylphosphat sowie verschiedene Hydroxy-TCP-Metabolite nachgewiesen werden. Es wird gemäß des NTP (National Toxicology Program) Berichts auf eine Beteiligung von (hepatischen) Oxidasen beim Abbau von TCP geschlossen (National Toxicology Program, Technical Report Series, No. 433, Toxicology and Carconogenesis - Studies of Tricresyl phosphate, 1994, NIH publication No. 94-3164, siehe https://ntp.niehs.nih.gov/ntp/htdocs/It_rpts/tr433.pdf).

Neben einer akuten Wirkung kommt auch eine chronische und akutchronische Wirkung als Ursache und Auslöser des Aerotoxischen Syndroms in Betracht.

Bisher im Stand der Technik bekannte Verfahren zum Nachweis eines Aerotoxischen Syndroms scheitern an der Halbwertzeit des auslösenden Trikresylphosphats, sowie am personellen und technisch aufwändigen Nachweis. Da es Flugpersonal nach Exposition mit Trikresylphosphat oft nicht zeitnah möglich ist, sich in medizinische Betreuung zu begeben, kann ein positiver Nachweis von Trikresylphosphat und/oder seiner Metabolite im Urin nach einem sog. 'fume event' (verstärkte Freisetzung von TCP in der Flugkabine) mit den bekannten Verfahren nur schwer bis gar nicht geführt werden. Somit steht gegenwärtig keine sichere diagnostische Methode zum Nachweis eines Aerotoxischen Syndroms zur Verfügung.

Ausgehend hiervon war es die Aufgabe der vorliegenden Erfindung ein in-vitro-Verfahren zur Diagnose des Risikos einer Person zur Ausbildung eines Aerotoxischen Syndroms und einen Kit zur Durchführung dieses Verfahrens bereitzustellen.

Die Aufgabe wird gelöst durch das Verfahren mit den Merkmalen von Anspruch 1 und dem Kit mit den Merkmalen von Anspruch 15. Die abhängigen Ansprüche zeigen vorteilhafte Weiterbildungen auf.

Erfindungsgemäß wird ein in-vitro-Verfahren zur Diagnose des Risikos einer Person zur Ausbildung eines Aerotoxischen Syndroms bereitgestellt, umfassend die Schritte
a) Untersuchen einer Probe einer Person mit einem molekulargenetischen Verfahren, wobei durch die Untersuchung bestimmt wird, ob mindestens ein Gen ausgewählt aus der Gruppe bestehend aus Genen, die für Butyrylcholinesterase, Cytochrom P450 2D6 und Cytochrom P450 2C9 kodieren, mindestens eine homozygote Sequenzvariante oder mindestens zwei heterozygote Sequenzvarianten aufweist, die jeweils dazu führen, dass Butyrylcholinesterase eine geringere Enzymaktivität als der Genotyp "GG" (optional: eine geringere Enzymaktivität als der Genotyp: "AG") aufweist und Cytochrom P450 2D6 und Cytochrom P450 2C9 eine geringere Enzymaktivität als der Phänotyp "extensiver Metabolisierer" (optional: eine geringe Enzymaktivität als der Phänotyp: "intermediärer Metabolisierer") aufweist, und
b) Einstufen der Person als anfällig, ein Aerotoxisches Syndrom zu entwickeln, wenn das mindestens eine Gen die mindestens eine homozygote Sequenzvariante oder die mindestens zwei heterozygoten Sequenzvarianten aufweist.

Es wurde gefunden, dass ein genetisch bedingter, beeinträchtigter Abbau von Trikresylphosphat zu neurotoxischen Beschwerden führt und Ursache des Aerotoxischen Syndroms ist. Als hierfür verantwortliche Gene wurden die drei Gene identifiziert, die für die Enzyme Butyrylcholinesterase, Cytochrom P450 2D6 und Cytochrom P450 2C9 kodieren. Mindestens eine homozygote Sequenzvariante oder mindestens zwei heterozygote Sequenzvarianten in einem dieser Gene, welche die Enzymaktivität gegenüber einer bestimmten phänotypischen Aktivität für jedes dieser Enzyme herabsetzt, führt zu einem deutlich reduzierten bis gänzlich fehlenden Abbau von Trikresylphosphat. Die Folge ist eine erhöhte Anfälligkeit, d.h. ein erhöhtes Risiko, nach Exposition mit TCP ein Aerotoxisches Syndrom zu entwickeln.

Das erfindungsgemäße Verfahren hat den Vorteil, dass es unabhängig von einer vorangegangenen Exposition einer Person mit TCP zur Diagnose des Risikos einer Person zur Ausbildung eines Aerotoxischen Syndroms verwendet werden kann. Anders ausgedrückt ist der Zeitpunkt der Untersuchung der Person unabhängig von einer TCP-Exposition frei wählbar. Zudem kann bereits vor einer Exposition mit TCP das (genetisch bedingte) Risiko einer Person erfasst werden, nach einer Exposition mit TCP ein Aerotoxisches Syndrom zu entwickeln. Faktoren wie beispielsweise die Einnahme bestimmter Medikamente, Konsum von Genussmitteln, Einnahme von Drogen o.ä. haben keinen Einfluss auf das Untersuchungsergebnis, da diese den Genotyp der zu untersuchenden Person nicht verändern.

Bevorzugt kodiert das mindestens eine Gen für Butyrylcholinesterase (BChE). Menschen können eine Sequenzvariante des BChE-Gens tragen, welches BChE-Enzym mit einer geringeren katalytischen Aktivität bildet als ein vom Genotyp "GG" kodiertes BChE-Enzym. Eine Hemmung dieser ohnehin geringeren enzymatischen Aktivität dieser Sequenzvarianten von BChE durch TCP und/oder dessen Metabolit Salegenin führt zu einer reduzierten bis völlig fehlenden Spaltung von Acetylcholin. Eine Anreicherung von Acetylcholin führt zu neurologischen Störungen und neurotoxischen Wirkungen und ist mitverantwortlich für eine Anfälligkeit eines Menschen, ein Aerotoxisches Syndrom zu entwickeln. Mit anderen Worten erlaubt die Identifizierung dieser Sequenzvarianten von BChE eine treffende Einstufung einer Person als anfällig, ein Aerotoxisches Syndrom zu entwickeln. Optional wird durch die Untersuchung in dem erfindungsgemäßen Verfahren bestimmt, ob das Gen, das für Butyrylcholinesterase kodiert, mindestens eine homozygote Sequenzvariante oder mindestens zwei heterozygote Sequenzvarianten aufweist, die jeweils dazu führt bzw. führen, dass Butyrylcholinesterase eine geringere Enzymaktivität als der Genotyp "AG" aufweist.

Das erfindungsgemäße Verfahren kann dadurch gekennzeichnet sein, dass durch die Untersuchung bestimmt wird, ob mindestens zwei Gene ausgewählt aus der Gruppe bestehend aus Genen, die für Butyrylcholinesterase, Cytochrom P450 2D6 und Cytochrom P450 2C9 kodieren, jeweils mindestens eine homozygote Sequenzvariante oder mindestens zwei heterozygote Sequenzvarianten aufweisen, die jeweils dazu führen, dass Butyrylcholinesterase eine geringere Enzymaktivität als der Genotyp "GG" (optional: geringer als der Genotyp "AG") aufweist und Cytochrom P450 2D6 und Cytochrom P450 2C9 eine geringere Enzymaktivität als der Phänotyp "extensiver Metabolisierer" (optional: geringer als der Phänotyp: "intermediärer Metabolisierer") aufweist, und die Person als anfällig, ein Aerotoxisches Syndrom zu entwickeln, eingestuft wird, wenn mindestens eines der mindestens zwei Gene, oder jeweils zwei der mindestens zwei Gene, die mindestens eine homozygote Sequenzvariante oder die mindestens zwei heterozygote Sequenzvarianten aufweist.

Bevorzugt kodiert von den mindestens zwei Genen mindestens ein Gen für Butyrylcholinesterase und mindestens ein Gen für Cytochrom P450 2D6.

In Bezug auf die Enzymaktivität von Cytochrom P450 2D6 gibt es ultraschnelle Metabolisierer, extensive (normale) Metabolisierer, intermediäre Metabolisierer und schlechte Metabolisierer, wobei die intermediären und schlechten Metabolisierer Trikresylphosphat deutlich langsamer abbauen. Es kommt somit bei diesen Phänotypen zu einer fehlenden Hydroxylierung und einer Akkumulation von TCP im menschlichen Organismus, was zur Steigerung der TCP Toxizität führt. Folglich ist durch die Identifizierung von Sequenzvarianten von Cytochrom P450 2D6, die den Phänotyp des intermediären oder schlechten Metabolisierers bewirken, eine noch treffendere Einstufung möglich, dass eine Anfälligkeit vorliegt, ein Aerotoxisches Syndrom zu entwickeln.

Das erfindungsgemäße Verfahren kann ferner dadurch gekennzeichnet sein, dass durch die Untersuchung bestimmt wird, ob mindestens drei Gene ausgewählt aus der Gruppe bestehend aus Genen, die für Butyrylcholinesterase, Cytochrom P450 2D6 und Cytochrom P450 2C9 kodieren, jeweils mindestens eine homozygote Sequenzvariante oder mindestens zwei heterozygote Sequenzvarianten aufweisen, die jeweils dazu führen, dass Butyrylcholinesterase eine geringere Enzymaktivität als der Genotyp "GG" (optional: geringer als der Genotyp: "AG") aufweist und Cytochrom P450 2D6 und Cytochrom P450 2C9 eine geringere Enzymaktivität als der Phänotyp "extensiver Metabolisierer" (optional: geringer als der Phänotyp: "intermediärer Metabolisierer") aufweist, und die Person als anfällig, ein Aerotoxisches Syndrom zu entwickeln, eingestuft wird, wenn mindestens eines der mindestens drei Gene, mindestens jeweils zwei der mindestens drei Gene, oder jeweils drei der mindestens drei Gene, die mindestens eine homozygote Sequenzvariante oder die mindestens zwei heterozygote Sequenzvarianten aufweist.

In Bezug auf die Enzymaktivität von Cytochrom P450 2C9 gibt es ultraschnelle Metabolisierer, extensive Metabolisierer, intermediäre Metabolisierer und schlechte Metabolisierer. Es wurde gefunden, dass Menschen mit Sequenzvarianten in dem Cytochrom P450 2C9 Gen, die zu einem intermediären oder schlechten Metabolisierer führen, Trikresylphosphat deutlich langsamer abbauen. Es kommt somit zu einer fehlenden Hydroxylierung von TCP und einer Akkumulation im menschlichen Organismus. Dies führt zu einer Steigerung der TCP-Toxizität und erhöht damit die Anfälligkeit eines Menschen, ein Aerotoxisches Syndrom zu entwickeln. Somit ist durch Identifizierung von Sequenzvarianten von Cytochrom P450 2C9, die den Phänotyp des intermediären oder schlechten Metabolisierers bewirken, eine weitere Steigerung der Genauigkeit der Einstufung möglich, dass eine Anfälligkeit vorliegt, ein Aerotoxisches Syndrom zu entwickeln.

Das molekulargenetische Verfahren kann ausgewählt sein aus der Gruppe bestehend aus DNA-Amplifikation mit anschließendem Restriktionsendonuklease-Verdau des Amplifikationsprodukts, DNA-Sequenzierung, RNA-Sequenzierung, Protease-Verdau mit anschließender Masseanalyse der verdauten Produkte Mischungen hiervon, bevorzugt ausgewählt aus der Gruppe bestehend aus Sanger-DNA-Sequenzierung, "Next-Generation"-Sequenzierung, Hybridisierung, PCR-RFLP, allelspezifische PCR, Pyrosequenzierung, "Molecular Beacon", Array-basierte Verfahren, RT-RNA-Amplifikation-Sequenzierung, MALDI-TOF-MS verdauter Proteine, ESI-MS verdauter Proteine.

Die Probe der Person kann ausgewählt sein aus der Gruppe bestehend aus Zellprobe, Körperflüssigkeitsprobe und Mischungen hiervon, bevorzugt ausgewählt aus der Gruppe bestehend aus Blut, Speichel, Aszites, Lymphe und Mischungen hiervon.

In einer bevorzugten Ausgestaltungsform enthält die Probe der Person DNA, die für ein Enzym ausgewählt aus der Gruppe bestehend aus Butyrylcholinesterase, Cytochrom P450 2D6, Cytochrom P450 2C9 und Kombinationen hiervon kodiert. In diesem Fall wird die DNA der Probe mit einem molekulargenetischen Verfahren untersucht. Die Probe enthält bevorzugt DNA, die für Butyrylcholinesterase kodiert, besonders bevorzugt DNA, die für Butyrylcholinesterase und Cytochrom P450 2D6 kodiert, insbesondere DNA, die für Butyrylcholinesterase, Cytochrom P450 2D6 und Cytochrom P450 2C9 kodiert.

In einer weiteren bevorzugten Ausführungsform enthält die Probe der Person mRNA, die für ein Enzym ausgewählt aus der Gruppe bestehend aus Butyrylcholinesterase, Cytochrom P450 2D6, Cytochrom P450 2C9 und Kombinationen hiervon kodiert. In diesem Fall wird die mRNA der Probe mit einem molekulargenetischen Verfahren untersucht. Die Probe enthält bevorzugt mRNA, die für Butyrylcholinesterase kodiert, besonders bevorzugt mRNA, die für Butyrylcholinesterase kodiert und mRNA, die für Cytochrom P450 2D6 kodiert, insbesondere mRNA, die für Butyrylcholinesterase kodiert, mRNA, die für Cytochrom P450 2D6 kodiert und mRNA, die für Cytochrom P450 2C9 kodiert.

Die Probe der Person kann ein Enzym enthalten, das ausgewählt ist aus der Gruppe bestehend aus Butyrylcholinesterase, Cytochrom P450 2D6, Cytochrom P450 2C9 und Kombinationen hiervon. In diesem Fall wird das Enzym der Probe mit einem molekulargenetischen Verfahren untersucht. Die Probe enthält bevorzugt Butyrylcholinesterase, besonders bevorzugt Butyrylcholinesterase und Cytochrom P450 2D6, insbesondere Butyrylcholinesterase, Cytochrom P450 2D6 und Cytochrom P450 2C9.

Die mindestens eine Sequenzvariante bei Butyrylcholinesterase kann die A- oder K-Sequenzvariante von Butyrylcholinesterase sein, bevorzugt ist es eine Sequenzvariante, die für eine Butyrylcholinesterase kodiert, die ausgewählt ist aus der Gruppe bestehend aus Asp70Gly, Ala539Thr und Kombinationen hiervon. Natürlich kommen hierfür auch andere funktionseinschränkende Sequenzvarianten in Betracht, die für eine reduzierte oder fehlende Aktivität der Butyrylcholinesterase verantwortlich sind (d.h. zu einer geringeren Enzymaktivität als der Genotyp "GG" führen).

Die mindestens eine Sequenzvariante bei Cytochrom P450 2D6 kann eine Sequenzvariante sein, die für ein Cytochrom P450 2D6 kodiert, das ausgewählt ist aus der Gruppe bestehend aus 259frameshift, IVSDS3, CYP2D6del, 118frameshift, His324Pro, Lys281del, Pro34Ser und Kombinationen hiervon. Natürlich kommen hierfür auch andere funktionseinschränkende Sequenzvarianten in Betracht, die für eine reduzierte oder fehlende Aktivität des Cytochrom P450 2D6 verantwortlich sind (d.h. zu einer geringeren Enzymaktivität als der Phänotyp "extensiver Metabolisierer" führen).

Die mindestens eine Sequenzvariante bei Cytochrom P450 2C9 kann die CYP2C9*3-Sequenzvariante von Cytochrom P450 2C9 sein, bevorzugt ist es eine Sequenzvariante, die für ein Cytochrom P450 2D6 Ile359Leu kodiert. Natürlich kommen hierfür auch andere funktionseinschränkende Sequenzvarianten in Betracht, die für eine reduzierte oder fehlende Aktivität des Cytochrom P450 2C9 verantwortlich sind (d.h. zu einer geringeren Enzymaktivität als der Phänotyp "extensiver Metabolisierer" führen).

Ferner kann im Rahmen des erfindungsgemäßen Verfahrens (in-vitro) diagnostiziert werden, dass die Person akut von einem Aerotoxischen Syndrom betroffen ist, wenn die Person mindestens eine Beschwerde zeigt, die typisch für ein Aerotoxisches Syndrom ist, bevorzugt eine neurotoxische Beschwerde, besonders bevorzugt eine neurotoxische Beschwerde ausgewählt aus der Gruppe bestehend aus Kopfschmerzen, Schwindel, Übelkeit und Kombinationen hiervon.

Ferner wird ein Kit zur Durchführung des erfindungsgemäßen in-vitro-Verfahrens bereitgestellt. Der Kit enthält Mittel zur Durchführung des erfindungsgemäßen in-vitro-Verfahrens.

Die Mittel zur Durchführung des erfindungsgemäßen in-vitro-Verfahrens können einen Array zur Aufnahme der Probe der Person umfassen.

Ferner können diese Mittel mindestens ein Primerpaar zur Amplifikation zumindest eines Bereichs mindestens eines Gens kodierend für Butyrylcholinesterase, Cytochrom P450 2D6 und/oder Cytochrom P450 2C9 umfassen.

Das mindestens eine Primerpaar kann zur Amplifikation zumindest eines Bereichs des BChE-Gens geeignet sein und bevorzugt mindestens eines der folgenden Primerpaare enthalten oder daraus bestehen:
▪ Primer mit SEQ-ID NO. 1 und Primer mit SEQ-ID NO. 2;
▪ Primer mit SEQ-ID NO. 3 und Primer mit SEQ-ID NO. 4;
▪ Primer mit SEQ-ID NO. 5 und Primer mit SEQ-ID NO. 6; und
▪ Primer mit SEQ-ID NO. 7 und Primer mit SEQ-ID NO. 8

Das mindestens eine Primerpaar kann zur Amplifikation zumindest eines Bereichs des CYP2D6-Gens geeignet sein und bevorzugt mindestens eines der folgenden Primerpaare enthalten oder daraus bestehen:
▪ Primer mit SEQ-ID NO. 11 und Primer mit SEQ-ID NO. 12;
▪ Primer mit SEQ-ID NO. 13 und Primer mit SEQ-ID NO. 14;
▪ Primer mit SEQ-ID NO. 15 und Primer mit SEQ-ID NO. 16; und
▪ Primer mit SEQ-ID NO. 17 und Primer mit SEQ-ID NO. 18.

Das mindestens eine Primerpaar kann zur Amplifikation zumindest eines Bereichs des CYP2C9-Gens geeignet sein und bevorzugt mindestens eines der folgenden Primerpaare enthalten oder daraus bestehen:
▪ Primer mit SEQ-ID NO. 26 und Primer mit SEQ-ID NO. 27;
▪ Primer mit SEQ-ID NO. 28 und Primer mit SEQ-ID NO. 29;
▪ Primer mit SEQ-ID NO. 30 und Primer mit SEQ-ID NO. 31;
▪ Primer mit SEQ-ID NO. 32 und Primer mit SEQ-ID NO. 33;
▪ Primer mit SEQ-ID NO. 34 und Primer mit SEQ-ID NO. 35;
▪ Primer mit SEQ-ID NO. 36 und Primer mit SEQ-ID NO. 37;
▪ Primer mit SEQ-ID NO. 38 und Primer mit SEQ-ID NO. 39;
▪ Primer mit SEQ-ID NO. 40 und Primer mit SEQ-ID NO. 41; und
▪ Primer mit SEQ-ID NO. 42 und Primer mit SEQ-ID NO. 43.

Darüberhinaus können diese Mittel mindestens einen weiteren Primer zur Sequenzierung zumindest eines Bereichs mindestens eines Gens kodierend für Butyrylcholinesterase, Cytochrom P450 2D6 und/oder Cytochrom P450 2C9 umfassen.

Der mindestens eine weitere Primer kann zur Sequenzierung zumindest eines Bereichs des BChE-Gens geeignet sein. Bevorzugt enthält der mindestens eine weitere Primer mindestens einen der folgenden Primer oder besteht daraus:
▪ Primer mit SEQ-ID NO. 2;
▪ Primer mit SEQ-ID NO. 4;
▪ Primer mit SEQ-ID NO. 5;
▪ Primer mit SEQ-ID NO. 6;
▪ Primer mit SEQ-ID NO. 9; und
▪ Primer mit SEQ-ID NO. 10.

Der mindestens eine weitere Primer kann zur Sequenzierung zumindest eines Bereichs des CYP2D6-Gens geeignet sein. Bevorzugt enthält der mindestens eine weitere Primer mindestens einen der folgenden Primer oder besteht daraus:
▪ Primer mit SEQ-ID NO. 13;
▪ Primer mit SEQ-ID NO. 15;
▪ Primer mit SEQ-ID NO. 19;
▪ Primer mit SEQ-ID NO. 20;
▪ Primer mit SEQ-ID NO. 21;
▪ Primer mit SEQ-ID NO. 22;
▪ Primer mit SEQ-ID NO. 23;
▪ Primer mit SEQ-ID NO. 24; und
▪ Primer mit SEQ-ID NO. 25.

Der mindestens eine weitere Primer kann zur Sequenzierung zumindest eines Bereichs des CYP2C9-Gens geeignet sein. Bevorzugt enthält der mindestens eine weitere Primer mindestens einen der folgenden Primer oder besteht daraus:
▪ Primer mit SEQ-ID NO. 26;
▪ Primer mit SEQ-ID NO. 28;
▪ Primer mit SEQ-ID NO. 30;
▪ Primer mit SEQ-ID NO. 32;
▪ Primer mit SEQ-ID NO. 34;
▪ Primer mit SEQ-ID NO. 36;
▪ Primer mit SEQ-ID NO. 38;
▪ Primer mit SEQ-ID NO. 40; und
▪ Primer mit SEQ-ID NO. 42.

Anhand der nachfolgenden Figuren soll der erfindungsgemäße Gegenstand näher erläutert werden, ohne diesen auf die hier dargestellten, spezifischen Ausführungsformen einschränken zu wollen.

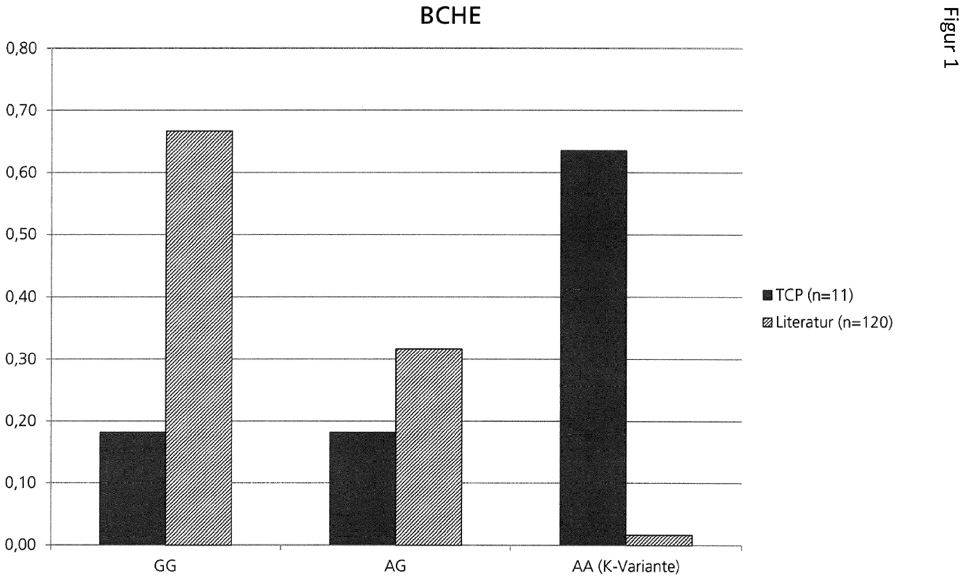

Figur 1 zeigt in einem Balkendiagramm die relative Häufigkeit, mit der Patienten an einem Aerotoxischen Syndrom erkrankt sind in Abhängigkeit von ihrem BChE-Genotyp und im Vergleich mit der Häufigkeit des jeweiligen Genotyps in der Bevölkerung ("GG" = GG-Genotyp, "AG" = AG-Genotyp, "AA" = AA-Genotyp). Es zeigte sich, dass Menschen mit dem AA-Genotyp (K-Variante) von BChE gegenüber einem rein statistischem Auftreten dieser Sequenzvariante signifikant häufiger eine Anfälligkeit zeigten, ein Aerotoxisches Syndrom zu entwickeln (p<0,001).

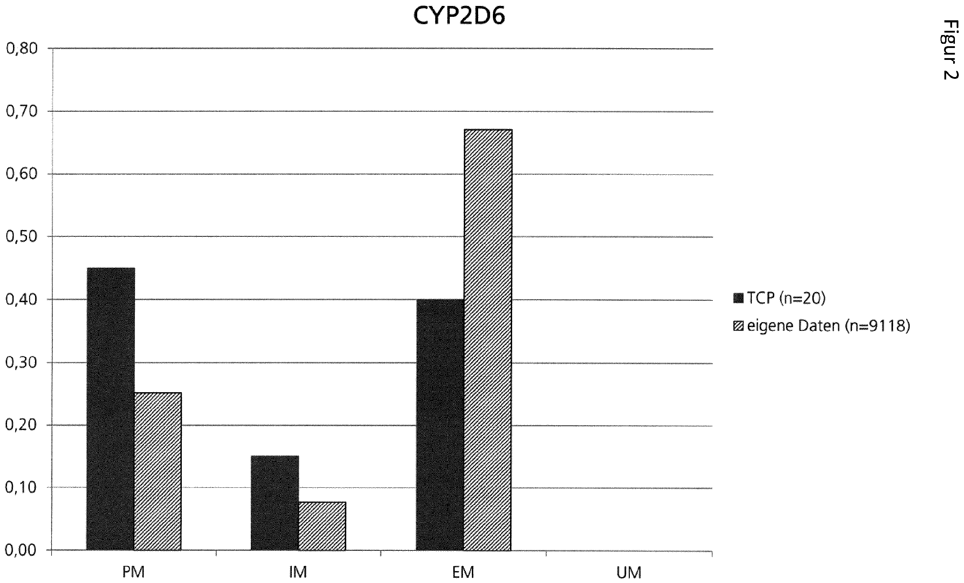

Figur 2 zeigt in einem Balkendiagramm die relative Häufigkeit, mit der Patienten an einem Aerotoxischen Syndrom erkrankt sind in Abhängigkeit von ihrem CYP2D6-Genotyp und im Vergleich mit der Häufigkeit des jeweiligen Genotyps in der Bevölkerung ("PM" = schlechter Metabolisierer, "IM" = intermediärer Metabolisierer", "EM" = extensiver Metabolisierer, "UM" = ultraschneller Metabolisierer). Es zeigte sich, dass Menschen mit einer Sequenzvariante von CYP2D6, die einen intermediären oder schlechten Metabolisierer bewirkt, gegenüber einem rein statistischem Auftreten dieser Sequenzvariante signifikant häufiger eine Anfälligkeit zeigten, ein Aerotoxisches Syndrom zu entwickeln (p<0,004).

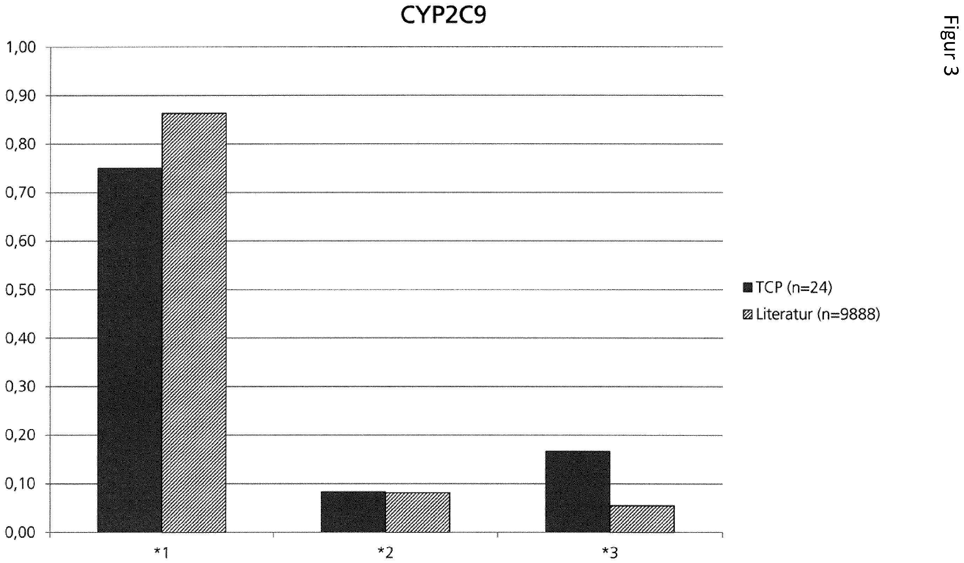

Figur 3 zeigt in einem Balkendiagramm die relative Häufigkeit, mit der Patienten an einem Aerotoxischen Syndrom erkrankt sind in Abhängigkeit von ihrem CYP2C9-Genotyp und im Vergleich mit der Häufigkeit des jeweiligen Genotyps in der Bevölkerung ("*1" = CYP2C9*1 Sequenzvariante, "*2" = CYP2C9*2 Sequenzvariante, "*3" = CYP2C9*3 Sequenzvariante). Es zeigte sich, dass Menschen mit einer Sequenzvariante von CYP2C9, die einen intermediären Metabolisierer bewirkt (CYP2C9*3 Sequenzvariante) gegenüber einem rein statistischem Auftreten dieser Sequenzvariante signifikant häufiger eine Anfälligkeit zeigten, ein Aerotoxisches Syndrom zu entwickeln (p<0,049).

### 1. Beispiel - Einfluss bestimmter Sequenzvarianten von BChE

Butyrylcholinesterase (BChE) spaltet Neurotransmitter (z.B. Acetylcholin). Eine Anreicherung von Acetylcholin führt zu neurologischen Störungen. TCP hat die Eigenschaft, Acetylcholinesterasen und BChE zu hemmen.

Die K-Sequenzvariante von BChE ist eine genetische BChE-Variante, die zu einer reduzierten Enzymaktivität von BChE führt, was die Spaltungsgeschwindigkeit von Acetylcholin negativ beeinflusst (geringere enzymatische Aktivität).

Menschen, die bestimmte Sequenzvarianten im BChE Gen tragen, bilden BChE Enzyme mit einer reduzierten katalytischen Aktivität. Eine Hemmung von BChE durch TCP und/oder dessen Metabolit Salegenin führt zu einer reduzierten bis völlig fehlenden Spaltung von Acetylcholin, was eine neurotoxische Wirkung ausübt.

In einer Gruppe von Menschen, welche einem Trikresylphosphat-haltigen Gas ausgesetzt wurden zeigte sich, dass gegenüber einer rein statistischen Häufigkeit der K-Sequenzvariante von BChE Menschen mit dieser Sequenzvariante signifikant häufiger ein Aerotoxisches Syndrom entwickelten (p<0,001; siehe Figur 1).

Als Probe für die Untersuchung des Genotyps der Personen wurde eine Blutprobe der Personen verwendet, die DNA der jeweiligen Personen enthielt. Zur Amplifikation der für die Untersuchung relevanten Genabschnitte des BChE-Gens wurden die folgenden Primerpaare bestehend aus Vorwärtsprimer (engl.: "forward primer") und Rückwärtsprimer (engl.: "reverse primer") eingesetzt ("F" steht für Vorwärtsprimer und "R" steht für Rückwärtsprimer):

**1. Primerpaar**

| | | |
|---|---|---|
| BChE 2.1F | gtaggcctttacagaagcag | (SEQ-ID NO. 1) |
| BChE 2.2R | tctggtgaacaatgaatggc | (SEQ-ID NO. 2) |

**2. Primerpaar**

| | | |
|---|---|---|
| BChE 2.3F | gagaaagtgcaggagcag | (SEQ-ID NO. 3) |
| BChE 2.4R | agaccaagcaaagctaagc | (SEQ-ID NO. 4) |

**3. Primerpaar**

| | | |
|---|---|---|
| BChE 3F | accactaagcccagttcac | (SEQ-ID NO. 5) |
| BChE 3R | tcagagatacatatagtaactcc | (SEQ-ID NO. 6) |

**4. Primerpaar**

| | | |
|---|---|---|
| BChE 4F | ctgtgtagttagagaaaatggc | (SEQ-ID NO. 7) |
| BChE 4R | gaaagaaattgaaccaggcc | (SEQ-ID NO. 8) |

Nach der Amplifikation der für die Untersuchung relevanten Genabschnitte des BChE-Gens wurden die Amplifikationsprodukte (amplifizierte DNA) über ein Sanger-Sequenzierungsverfahren sequenziert. Für die Sequenzierung wurden die folgenden Primer eingesetzt:

| | | |
|---|---|---|
| BChE 2.1R | ggttccacatctctgatcc | (SEQ-ID NO. 9) |
| BChE 2.2R | tctggtgaacaatgaatggc | (SEQ-ID NO. 2) |
| BChE 2.3R | tctacccagtctgtgtaatg | (SEQ-ID NO. 10) |
| BChE 2.4R | agaccaagcaaagctaagc | (SEQ-ID NO. 4) |
| BChE 3F | accactaagcccagttcac | (SEQ-ID NO. 5) |
| BChE 4R | gaaagaaattgaaccaggcc | (SEQ-ID NO. 6) |

### 2. Beispiel - Einfluss bestimmter Sequenzvarianten von CYP2D6

CYP2D6 Enzyme sind am Abbau zahlreicher Fremdstoffe beteiligt. Der Abbau CYP2D6-abhängiger Medikamente kann zum Auftreten unerwünschter Arzneimittelwirkungen führen.

Im Falle des CYP2D6 Enzyms sind Sequenzvarianten bekannt, die zu einem sog. schlechten Metabolisierer, einem sog. intermediären Metabolisierer, einem sog. extensiven Metabolisierer und einem sog. ultraschnellen Metabolisierer in Bezug auf die Verstoffwechslung CYP2D6-abhängiger Medikamente führen.

Es wurde gefunden, dass schlechte Metabolisierer und intermediäre Metabolisierer das Trikresylphosphat deutlich langsamer abbauen. Es kommt somit zu einer fehlenden Hydroxylierung und einer Akkumulation von TCP im menschlichen Organismus, was zur Steigerung der TCP Toxizität führt.

In einer Gruppe von Menschen, welche einem Trikresylphosphat-haltigen Gas ausgesetzt wurden zeigte sich, dass gegenüber einer rein statistischen Häufigkeit des intermediären Metabolisierers und schlechten Metabolisieres Menschen mit dieser Sequenzvariante signifikant häufiger ein Aerotoxisches Syndrom entwickelten ist (p<0,004; siehe Figur 2).

Gegenwärtig sind mehr als 100 Sequenzvarianten im CYP2D6 Gen dokumentiert. Die Erfindung beruht auf dem Nachweis mindestens einer CYP2D6 Genvariante in homozygot oder zwei CYP2D6 Genvarianten in heterozygoter Form, die zu einer Verschlechterung der Aktivität des CYP2D6 Enzyms führen.

Als Probe für die Untersuchung des Genotyps der Personen wurde eine Blutprobe der Personen verwendet, die DNA der jeweiligen Personen enthielt. Zur Amplifikation der für die Untersuchung relevanten Genabschnitte des CYP2D6-Gens wurden die folgenden Primerpaare bestehend aus Vorwärtsprimer (engl.: "forward primer") und Rückwärtsprimer (engl.: "reverse primer") eingesetzt ("F" steht für Vorwärtsprimer und "R" steht für Rückwärtsprimer):

**1. Primerpaar**

| | | |
|---|---|---|
| CYP2D6 1f5 | cagtcaacacagcaggttc | (SEQ-ID NO. 11) |
| CYP2D6 1r5 | cctgtttcatgtccacgac | (SEQ-ID NO. 12) |

**2. Primerpaar**

| | | |
|---|---|---|
| CYP2D6 2F | atgaaacaggccagcgagt | (SEQ-ID NO. 13) |
| CYP2D6 2R | CTGACGTGGATAGGAGGTA | (SEQ-ID NO. 14) |

**3. Primerpaar**

| | | |
|---|---|---|
| CYP2D6 3F | TACCTCCTATCCACGTCAG | (SEQ-ID NO. 15) |
| CYP2D6 3R | ccggccctgacactccttc | (SEQ-ID NO. 16) |

**4. Primerpaar**

| | | |
|---|---|---|
| CYP2D6 4F | caacataggaggcaagaag | (SEQ-ID NO. 17) |
| CYP2D6 5R3 | atatagctccctgacgcc | (SEQ-ID NO. 18) |

Nach der Amplifikation der für die Untersuchung relevanten Genabschnitte des CYP2D6-Gens wurden die Amplifikationsprodukte (amplifizierte DNA) über ein Sanger-Sequenzierungsverfahren sequenziert. Für die Sequenzierung wurden die folgenden Primer eingesetzt:

| | | |
|---|---|---|
| CYP2D6 1ss | tctgggaatgggatgctaac | (SEQ-ID NO. 19) |
| CYP2D6 201 | aaatgcccttctccaggaagt | (SEQ-ID NO. 20) |
| CYP2D6 2F | atgaaacaggccagcgagt | (SEQ-ID NO. 13) |
| CYP2D6 2s | tgatgggcagaagggcac | (SEQ-ID NO. 21) |
| CYP2D6 204 | agaccagggggagcatagggtt | (SEQ-ID NO. 22) |
| CYP2D6 3F | TACCTCCTATCCACGTCAG | (SEQ-ID NO. 15) |
| CYP2D6 3s | tgacaggtgcagaattggag | (SEQ-ID NO. 23) |
| CYP2D6 4fs | aggcaagaaggagtgtcag | (SEQ-ID NO. 24) |
| CYP2D6 4s | tgccagaatgttggaggac | (SEQ-ID NO. 25) |

### 3. Beispiel - Einfluss bestimmter Sequenzvarianten von CYP2C9

CYP2C9 Enzyme sind am Abbau zahlreicher Fremdstoffe beteiligt. Der Abbau CYP2C9-abhängiger Medikamente kann zum Auftreten unerwünschter Arzneimittelwirkungen führen.

Es wurde gefunden, dass schlechte Metabolisierer und intermediäre Metabolisierer das Trikresylphosphat deutlich langsamer abbauen. Es kommt somit zu einer fehlenden Hydroxylierung von TCP und einer Akkumulation im menschlichen Organismus, was zur Steigerung der TCP-Toxizität führt.

Die Sequenzvariante CYP2C9*3 führt zu einem intermediären Metabolisierer und, d.h. Trikresylphosphat wird durch das Enyzm dieser Sequenzvariante deutlich langsamer abgebaut als bei einem extensiven oder einem ultraschnellen Metabolisierer.

In einer Gruppe von Menschen, welche einem Trikresylphosphat-haltigen Gas ausgesetzt wurden zeigte sich, dass gegenüber einer rein statistischen Häufigkeit der intermediäre Metabolisierers mit der CYP2C9*3 Sequenzvariante signifikant häufiger ein Aerotoxisches Syndrom entwickelte (p<0,049; siehe Figur 3).

Als Probe für die Untersuchung des Genotyps der Personen wurde eine Blutprobe der Personen verwendet, die DNA der jeweiligen Personen enthielt. Zur Amplifikation der für die Untersuchung relevanten Genabschnitte des CYP2C9-Gens wurden die folgenden Primerpaare bestehend aus Vorwärtsprimer (engl.: "forward primer") und Rückwärtsprimer (engl.: "reverse primer") eingesetzt ("F" steht für Vorwärtsprimer und "R" steht für Rückwärtsprimer):

**1. Primerpaar**

| | | |
|---|---|---|
| CYP2C9 1F | catcaaagaggcacacaccg | (SEQ-ID NO. 26) |
| CYP2C9 1R2 | TTCTCTACTCACAAAATACATGG | (SEQ-ID NO. 27) |

**2. Primerpaar**

| | | |
|---|---|---|
| CYP2C9 2F1 | CAATGAAAATATCATGCTAAATCAG | (SEQ-ID NO. 28) |
| CYP2C9 2R | gaggagctctgtaagtctc | (SEQ-ID NO. 29) |

**3. Primerpaar**

| | | |
|---|---|---|
| CYP2C9 3F | gatggaaaacagagacttacag | (SEQ-ID NO. 30) |
| CYP2C9 3R | CAGGACTCATAATGAAAGATATG | (SEQ-ID NO. 31) |

**4. Primerpaar**

| | | |
|---|---|---|
| CYP2C9 4F | TAGGTTGTAATGGTCAACTCAG | (SEQ-ID NO. 32) |
| CYP2C9 4R | cacttcagagcttgatccatg | (SEQ-ID NO. 33) |

**5. Primerpaar**

| | | |
|---|---|---|
| CYP2C9 5F | ctggttagaattgatcctctg | (SEQ-ID NO.34) |
| CYP2C9 5R | caagcattactgattgaccag | (SEQ-ID NO. 35) |

**6. Primerpaar**

| | | |
|---|---|---|
| CYP2C9 6F2 | ctatgttggtaactatataactatgtg | (SEQ-ID NO. 36) |
| CYP2C9 6R2 | ATTACAGCTGGAAGCACAAAAG | (SEQ-ID NO. 37) |

**7. Primerpaar**

| | | |
|---|---|---|
| CYP2C9 7F | tcttaatatctggtttatggcag | (SEQ-ID NO. 38) |
| CYP2C9 7R1 | ACTATGAATTTGGGGACTTCG | (SEQ-ID NO. 39) |

**8. Primerpaar**

| | | |
|---|---|---|
| CYP2C9 8F | ttctttggaacgggatttcc | (SEQ-ID NO. 40) |
| CYP2C9 8R | GTATCATGAGCAGGGTGTAC | (SEQ-ID NO. 41) |

**9. Primerpaar**

| | | |
|---|---|---|
| CYP2C9 9f2 | tgtttgtgccagttacagag | (SEQ-ID NO. 42) |
| CYP2C9 9R | ctcaagtaactctaacactcac | (SEQ-ID NO. 43) |

Nach der Amplifikation der für die Untersuchung relevanten Genabschnitte des CYP2C9-Gens wurden die Amplifikationsprodukte (amplifizierte DNA) über ein Sanger-Sequenzierungsverfahren sequenziert. Für die Sequenzierung wurden die folgenden Primer eingesetzt:

| | | |
|---|---|---|
| CYP2C9 1F | catcaaagaggcacacaccg | (SEQ-ID NO. 26) |
| CYP2C9 2F1 | CAATGAAAATATCATGCTAAATCAG | (SEQ-ID NO. 28) |
| CYP2C9 3F | gatggaaaacagagacttacag | (SEQ-ID NO. 30) |
| CYP2C9 4F | TAGGTTGTAATGGTCAACTCAG | (SEQ-ID NO. 32) |
| CYP2C9 5F | ctggttagaattgatcctctg | (SEQ-ID NO. 34) |
| CYP2C9 6F2 | ctatgttggtaactatataactatgtg | (SEQ-ID NO. 36) |
| CYP2C9 7F | tcttaatatctggtttatggcag | (SEQ-ID NO. 38) |
| CYP2C9 8F | ttctttggaacgggatttcc | (SEQ-ID NO. 40) |
| CYP2C9 9f2 | tgtttgtgccagttacagag | (SEQ-ID NO. 42) |

## Patentansprüche

1. *In*-*vitro*-Verfahren zur Diagnose des Risikos einer Person zur Ausbildung eines Aerotoxischen Syndroms, umfassend die Schritte
a) Untersuchen einer Probe einer Person mit einem molekulargenetischen Verfahren, wobei durch die Untersuchung bestimmt wird, ob mindestens ein Gen ausgewählt aus der Gruppe bestehend aus Genen, die für Butyrylcholinesterase, Cytochrom P450 2D6 und Cytochrom P450 2C9 kodieren, mindestens eine homozygote Sequenzvariante oder mindestens zwei heterozygote Sequenzvarianten aufweist, die jeweils dazu führen, dass Butyrylcholinesterase eine geringere Enzymaktivität als der Genotyp "GG" aufweist und Cytochrom P450 2D6 und Cytochrom P450 2C9 eine geringere Enzymaktivität als der Phänotyp "extensiver Metabolisierer" aufweist, und
b) Einstufen der Person als anfällig, ein Aerotoxisches Syndrom zu entwickeln, wenn das mindestens eine Gen die mindestens eine homozygote Sequenzvariante oder die mindestens zwei heterozygoten Sequenzvarianten aufweist.

2. *In*-*vitro*-Verfahren gemäß Anspruch 1, **dadurch gekennzeichnet, dass** das mindestens eine Gen für Butyrylcholinesterase kodiert.

3. *In*-*vitro*-Verfahren gemäß einem der vorhergehenden Ansprüche, **dadurch gekennzeichnet, dass** durch die Untersuchung bestimmt wird, ob mindestens zwei Gene ausgewählt aus der Gruppe bestehend aus Genen, die für Butyrylcholinesterase, Cytochrom P450 2D6 und Cytochrom P450 2C9 kodieren, jeweils mindestens eine homozygote Sequenzvariante oder mindestens zwei heterozygote Sequenzvarianten aufweisen, die jeweils dazu führen, dass Butyrylcholinesterase eine geringere Enzymaktivität als der Genotyp "GG" aufweist und Cytochrom P450 2D6 und Cytochrom P450 2C9 eine geringere Enzymaktivität als der Phänotyp "extensiver Metabolisierer" aufweist, und die Person als anfällig, ein Aerotoxisches Syndrom zu entwickeln, eingestuft wird, wenn mindestens eines der mindestens zwei Gene, oder jeweils zwei der mindestens zwei Gene, die mindestens eine homozygote Sequenzvariante oder die mindestens zwei heterozygote Sequenzvarianten aufweist.

4. *In*-*vitro*-Verfahren gemäß dem vorhergehenden Anspruch, **dadurch gekennzeichnet, dass** von den mindestens zwei Genen mindestens ein Gen für Butyrylcholinesterase kodiert und mindestens ein Gen für Cytochrom P450 2D6 kodiert.

5. *In*-*vitro*-Verfahren gemäß einem der vorhergehenden Ansprüche, **dadurch gekennzeichnet, dass** durch die Untersuchung bestimmt wird, ob mindestens drei Gene ausgewählt aus der Gruppe bestehend aus Genen, die für Butyrylcholinesterase, Cytochrom P450 2D6 und Cytochrom P450 2C9 kodieren, jeweils mindestens eine homozygote Sequenzvariante oder mindestens zwei heterozygote Sequenzvarianten aufweisen, die jeweils dazu führen, dass Butyrylcholinesterase eine geringere Enzymaktivität als der Genotyp "GG" aufweist und Cytochrom P450 2D6 und Cytochrom P450 2C9 eine geringere Enzymaktivität als der Phänotyp "extensiver Metabolisierer" aufweist, und die Person als anfällig, ein Aerotoxisches Syndrom zu entwickeln, eingestuft wird, wenn mindestens eines der mindestens drei Gene, mindestens jeweils zwei der mindestens drei Gene, oder jeweils drei der mindestens drei Gene, die mindestens eine homozygote Sequenzvariante oder die mindestens zwei heterozygote Sequenzvarianten aufweist.

6. *In*-*vitro*-Verfahren zur Diagnose gemäß einem der vorhergehenden Ansprüche, **dadurch gekennzeichnet, dass** das molekulargenetische Verfahren ausgewählt ist aus der Gruppe bestehend aus DNA-Amplifikation mit anschließendem Restriktionsendonuklease-Verdau des Amplifikationsprodukts, DNA-Sequenzierung, RNA-Sequenzierung, Protease-Verdau mit anschließender Masseanalyse der verdauten Produkte Mischungen hiervon, bevorzugt ausgewählt aus der Gruppe bestehend aus Sanger-DNA-Sequenzierung, "Next-Generation"-Sequenzierung, Hybridisierung, PCR-RFLP, allelspezifische PCR, Pyrosequenzierung, "Molecular Beacon", Array-basierte Verfahren, RT-RNA-Amplifikation-Sequenzierung, MALDI-TOF-MS verdauter Proteine, ESI-MS verdauter Proteine.

7. *In*-*vitro*-Verfahren zur Diagnose gemäß einem der vorhergehenden Ansprüche, **dadurch gekennzeichnet, dass** die Probe der Person ausgewählt ist aus der Gruppe bestehend aus Zellprobe, Körperflüssigkeitsprobe und Mischungen hiervon, bevorzugt ausgewählt aus der Gruppe bestehend aus Blut, Speichel, Aszites, Lymphe und Mischungen hiervon.

8. *In*-*vitro*-Verfahren zur Diagnose gemäß einem der vorhergehenden Ansprüche, **dadurch gekennzeichnet, dass** die Probe der Person DNA enthält, die für ein Enzym ausgewählt aus der Gruppe bestehend aus Butyrylcholinesterase, Cytochrom P450 2D6, Cytochrom P450 2C9 und Kombinationen hiervon kodiert und die DNA der Probe mit einem molekulargenetischen Verfahren untersucht wird, wobei die Probe bevorzugt DNA enthält, die für Butyrylcholinesterase kodiert, besonders bevorzugt DNA, die für Butyrylcholinesterase und Cytochrom P450 2D6 kodiert, insbesondere DNA, die für Butyrylcholinesterase, Cytochrom P450 2D6 und Cytochrom P450 2C9 kodiert.

9. *In*-*vitro*-Verfahren zur Diagnose gemäß einem der vorhergehenden Ansprüche, **dadurch gekennzeichnet, dass** die Probe der Person mRNA enthält, die für ein Enzym ausgewählt aus der Gruppe bestehend aus Butyrylcholinesterase, Cytochrom P450 2D6, Cytochrom P450 2C9 und Kombinationen hiervon kodiert und die mRNA der Probe mit einem molekulargenetischen Verfahren untersucht wird, wobei die Probe bevorzugt mRNA enthält, die für Butyrylcholinesterase kodiert, besonders bevorzugt mRNA, die für Butyrylcholinesterase kodiert und mRNA, die für Cytochrom P450 2D6 kodiert, insbesondere mRNA, die für Butyrylcholinesterase kodiert, mRNA, die für Cytochrom P450 2D6 kodiert und mRNA, die für Cytochrom P450 2C9 kodiert.

10. *In*-*vitro*-Verfahren zur Diagnose gemäß einem der vorhergehenden Ansprüche, **dadurch gekennzeichnet, dass** die Probe der Person ein Enzym enthält, das ausgewählt ist aus der Gruppe bestehend aus Butyrylcholinesterase, Cytochrom P450 2D6, Cytochrom P450 2C9 und Kombinationen hiervon, und das Enzym der Probe mit einem molekulargenetischen Verfahren untersucht wird, wobei die Probe bevorzugt Butyrylcholinesterase, besonders bevorzugt Butyrylcholinesterase und Cytochrom P450 2D6, insbesondere Butyrylcholinesterase, Cytochrom P450 2D6 und Cytochrom P450 2C9, enthält.

11. *In*-*vitro*-Verfahren zur Diagnose gemäß einem der vorhergehenden Ansprüche, **dadurch gekennzeichnet, dass** die mindestens eine Sequenzvariante bei Butyrylcholinesterase die A- oder K-Sequenzvariante von Butyrylcholinesterase ist, bevorzugt eine Sequenzvariante, die für eine Butyrylcholinesterase kodiert, die ausgewählt ist aus der Gruppe bestehend aus Asp70Gly, Ala539Thr und Kombinationen hiervon sowie andere Sequenzvarianten, die den Phänotyp eines fehlenden BChEabhängigen Metabolismus bewirken.

12. *In*-*vitro*-Verfahren zur Diagnose gemäß einem der vorhergehenden Ansprüche, **dadurch gekennzeichnet, dass** die mindestens eine Sequenzvariante bei Cytochrom P450 2D6 eine Sequenzvariante ist, die für ein Cytochrom P450 2D6 kodiert, das ausgewählt ist aus der Gruppe bestehend aus 259frameshift, IVSDS3, CYP2D6del, 118frameshift, His324Pro, Lys281del, Pro34Ser und Kombinationen hiervon sowie andere Sequenzvarianten, die den Phänotyp eines intermediären oder schlechten Metabolisierers bewirken.

13. *In*-*vitro*-Verfahren zur Diagnose gemäß einem der vorhergehenden Ansprüche, **dadurch gekennzeichnet, dass** die mindestens eine Sequenzvariante bei Cytochrom P450 2C9 die CYP2C9*3-Sequenzvariante von Cytochrom P450 2C9 ist, bevorzugt eine Sequenzvariante, die für ein Cytochrom P450 2D6 Ile359Leu kodiert sowie andere Sequenzvarianten, die den Phänotyp eines intermediären oder schlechten Metabolisierers bewirken.

14. *In*-*vitro*-Verfahren zur Diagnose gemäß einem der vorhergehenden Ansprüche, **dadurch gekennzeichnet, dass** ferner diagnostiziert wird, dass die Person akut von einem Aerotoxischen Syndrom betroffen ist, wenn die Person mindestens eine Beschwerde zeigt, die typisch für ein Aerotoxisches Syndrom ist, bevorzugt eine neurotoxische Beschwerde, besonders bevorzugt eine neurotoxische Beschwerde ausgewählt aus der Gruppe bestehend aus Kopfschmerzen, Schwindel, Übelkeit und Kombinationen hiervon.

15. Kit zur Durchführung eines *In*-*vitro*-Verfahrens gemäß einem der folgenden Ansprüche, enthaltend Mittel zur Durchführung des *in-vitro-*Verfahrens gemäß einem der vorhergehenden Ansprüche, wobei diese Mittel bevorzugt einen Array zur Aufnahme einer Probe einer Person umfassen, besonders bevorzugt mindestens ein Primerpaar umfassen, das zur Amplifikation zumindest eines Bereichs mindestens eines Gens kodierend für Butyrylcholinesterase, Cytochrom P450 2D6 und/oder Cytochrom P450 2C9 geeignet ist, insbesondere mindestens einen weiteren Primer umfassen, der zur Sequenzierung zumindest eines Bereichs mindestens eines Gens kodierend für Butyrylcholinesterase, Cytochrom P450 2D6 und/oder Cytochrom P450 2C9 geeignet ist.


SEQUENZPROTOKOLL

<110> Institut für Pharmakogenetik und genetische Disposition

<120> In-vitro-Verfahren zur Diagnose des Risikos einer Person zur
Ausbildung eines Aerotoxisches Syndroms und Kit zur Durchführung des
Verfahrens

<130> 179EP 0314

<160> 43

<170> BiSSAP 1.3.6

<210> 1
<211> 20
<212> DNA
<213> künstliche Sequenz

<220>
<223> BChE 2.1F Primer

<400> 1
gtaggccttt acagaagcag                                                20

<210> 2
<211> 20
<212> DNA
<213> künstliche Sequenz

<220>
<223> BChE 2.2R Primer

<400> 2
tctggtgaac aatgaatggc                                                20

<210> 3
<211> 18
<212> DNA
<213> künstliche Sequenz

<220>
<223> BChE 2.3F Primer

<400> 3
gagaaagtgc aggagcag                                                  18

<210> 4
<211> 19
<212> DNA
<213> künstliche Sequenz

<220>
<223> BChE 2.4R Primer

<400> 4

agaccaagca aagctaagc 19

<210> 5
<211> 19
<212> DNA
<213> künstliche Sequenz

<220>
<223> BChE 3F Primer

<400> 5
accactaagc ccagttcac 19

<210> 6
<211> 23
<212> DNA
<213> künstliche Sequenz

<220>
<223> BChE 3R Primer

<400> 6
tcagagatac atatagtaac tcc 23

<210> 7
<211> 22
<212> DNA
<213> künstliche Sequenz

<220>
<223> BChE 4F Primer

<400> 7
ctgtgtagtt agagaaaatg gc 22

<210> 8
<211> 20
<212> DNA
<213> künstliche Sequenz

<220>
<223> BChE 4R Primer

<400> 8
gaaagaaatt gaaccaggcc 20

<210> 9
<211> 19
<212> DNA
<213> künstliche Sequenz

<220>
<223> BChE 2.1R Primer

<400> 9
ggttccacat ctctgatcc                                                    19


<210> 10
<211> 20
<212> DNA
<213> künstliche Sequenz


<220>
<223> BChE 2.3R Primer

<400> 10
tctacccagt ctgtgtaatg                                                   20


<210> 11
<211> 19
<212> DNA
<213> künstliche Sequenz


<220>
<223> CYP2D6 1f5 Primer

<400> 11
cagtcaacac agcaggttc                                                    19


<210> 12
<211> 19
<212> DNA
<213> künstliche Sequenz


<220>
<223> CYP2D6 1r5 Primer

<400> 12
cctgtttcat gtccacgac                                                    19


<210> 13
<211> 19
<212> DNA
<213> künstliche Sequenz


<220>
<223> CYP2D6 2F Primer

<400> 13
atgaaacagg ccagcgagt                                                    19


<210> 14
<211> 19
<212> DNA
<213> künstliche Sequenz

<220>
<223> CYP2D6 2R Primer

<400> 14
ctgacgtgga taggaggta                                                    19


<210> 15
<211> 19
<212> DNA
<213> künstliche Sequenz


<220>
<223> CYP2D6 3F Primer

<400> 15
tacctcctat ccacgtcag                                                    19


<210> 16
<211> 20
<212> DNA
<213> künstliche Sequenz


<220>
<223> CYP2D6 3R Primer

<400> 16
ccggccctga cactccttct                                                   20


<210> 17
<211> 19
<212> DNA
<213> künstliche Sequenz


<220>
<223> CYP2D6 4F Primer

<400> 17
caacatagga ggcaagaag                                                    19


<210> 18
<211> 18
<212> DNA
<213> künstliche Sequenz


<220>
<223> CYP2D6 5R3 Primer

<400> 18
atatagctcc ctgacgcc                                                     18


<210> 19
<211> 20
<212> DNA
<213> künstliche Sequenz

<220>
<223> CYP2D6 1ss Primer

<400> 19
tctgggaatg ggatgctaac                                                    20


<210> 20
<211> 21
<212> DNA
<213> künstliche Sequenz


<220>
<223> CYP2D6 201 Primer

<400> 20
aaatgccctt ctccaggaag t                                                  21


<210> 21
<211> 18
<212> DNA
<213> künstliche Sequenz


<220>
<223> CYP2D6 2s Primer

<400> 21
tgatgggcag aagggcac                                                      18


<210> 22
<211> 22
<212> DNA
<213> künstliche Sequenz


<220>
<223> CYP2D6 204 Primer

<400> 22
agaccagggg gagcataggg tt                                                 22


<210> 23
<211> 20
<212> DNA
<213> künstliche Sequenz


<220>
<223> CYP2D6 3s Primer

<400> 23
tgacaggtgc agaattggag                                                    20


<210> 24
<211> 19

<212> DNA
<213> künstliche Sequenz

<220>
<223> CYP2D6 4fs Primer

<400> 24
aggcaagaag gagtgtcag 19

<210> 25
<211> 19
<212> DNA
<213> künstliche Sequenz

<220>
<223> CYP2D6 4s Primer

<400> 25
tgccagaatg ttggaggac 19

<210> 26
<211> 20
<212> DNA
<213> künstliche Sequenz

<220>
<223> CYP2C9 1F Primer

<400> 26
catcaaagag gcacacaccg 20

<210> 27
<211> 23
<212> DNA
<213> künstliche Sequenz

<220>
<223> CYP2C9 1R2 Primer

<400> 27
ttctctactc acaaaataca tgg 23

<210> 28
<211> 25
<212> DNA
<213> künstliche Sequenz

<220>
<223> CYP2C9 2F1 Primer

<400> 28
caatgaaaat atcatgctaa atcag 25

<210> 29
<211> 19
<212> DNA
<213> künstliche Sequenz

<220>
<223> CYP2C9 2R Primer

<400> 29
gaggagctct gtaagtctc 19

<210> 30
<211> 22
<212> DNA
<213> künstliche Sequenz

<220>
<223> CYP2C9 3F Primer

<400> 30
gatggaaaac agagacttac ag 22

<210> 31
<211> 23
<212> DNA
<213> künstliche Sequenz

<220>
<223> CYP2C9 3R Primer

<400> 31
caggactcat aatgaaagat atg 23

<210> 32
<211> 22
<212> DNA
<213> künstliche Sequenz

<220>
<223> CYP2C9 4F Primer

<400> 32
taggttgtaa tggtcaactc ag 22

<210> 33
<211> 21
<212> DNA
<213> künstliche Sequenz

<220>
<223> CYP2C9 4R Primer

<400> 33
cacttcagag cttgatccat g 21

<210> 34
<211> 21
<212> DNA
<213> künstliche Sequenz

<220>
<223> CYP2C9 5F Primer

<400> 34
ctggttagaa ttgatcctct g                                                21

<210> 35
<211> 21
<212> DNA
<213> künstliche Sequenz

<220>
<223> CYP2C9 5R Primer

<400> 35
caagcattac tgattgacca g                                                21

<210> 36
<211> 27
<212> DNA
<213> künstliche Sequenz

<220>
<223> CYP2C9 6F2 Primer

<400> 36
ctatgttggt aactatataa ctatgtg                                          27

<210> 37
<211> 22
<212> DNA
<213> künstliche Sequenz

<220>
<223> CYP2C9 6R2 Primer

<400> 37
attacagctg gaagcacaaa ag                                               22

<210> 38
<211> 23
<212> DNA
<213> künstliche Sequenz

<220>
<223> CYP2C9 7F Primer

<400> 38
tcttaatatc tggtttatgg cag                                                23

<210> 39
<211> 21
<212> DNA
<213> künstliche Sequenz

<220>
<223> CYP2C9 7R1 Primer

<400> 39
actatgaatt tggggacttc g                                                  21

<210> 40
<211> 20
<212> DNA
<213> künstliche Sequenz

<220>
<223> CYP2C9 8F Primer

<400> 40
ttctttggaa cgggatttcc                                                    20

<210> 41
<211> 20
<212> DNA
<213> künstliche Sequenz

<220>
<223> CYP2C9 8R Primer

<400> 41
gtatcatgag cagggtgtac                                                    20

<210> 42
<211> 20
<212> DNA
<213> künstliche Sequenz

<220>
<223> CYP2C9 9f2 Primer

<400> 42
tgtttgtgcc agttacagag                                                    20

<210> 43
<211> 22
<212> DNA
<213> künstliche Sequenz

<220>

<223> CYP2C9 9R Primer

<400> 43
ctcaagtaac tctaacactc ac                                                22